# EUROPÄISCHE PATENTSCHRIFT

(11) **EP 1 505 981 B1**
(45) Veröffentlichungstag und Bekanntmachung des Hinweises auf die Patenterteilung: **11.07.2012**
(21) Anmeldenummer: 03727422.2
(22) Anmeldetag: 02.05.2003
(51) Int. Cl.: A61K 31/517, A61K 31/47, A61K 31/519, A61P 13/08

(54) **VERWENDUNG VON HEMMERN DER EGFR-VERMITTELTEN SIGNALTRANSDUKTION ZUR BEHANDLUNG VON GUTARTIGER PROSTATAHYPERPLASIE (BPH)/PROSTATAHYPERTROPHIE**
UTILIZATION OF INHIBITORS OF EGFR-MEDIATED SIGNAL TRANSDUCTION FOR THE TREATMENT OF BENIGN PROSTATIC HYPERPLASIA (BPH)/PROSTATIC HYPERTROPHY
UTILISATION D'INHIBITEURS DE LA TRANSDUCTION DE SIGNAL INDUITE PAR LE RECEPTEUR EGF DANS LE TRAITEMENT DE L'HYPERPLASIE/HYPERTROPHIE BENIGNE DE LA PROSTATE

(30) Priorität: 11.05.2002 DE 10221018
(43) Veröffentlichungstag der Anmeldung: 16.02.2005
(73) Patentinhaber: Boehringer Ingelheim Pharma GmbH & Co. KG, 55216 Ingelheim am Rhein (DE)
(72) Erfinder: SINGER, Thomas, 79594 Inzlingen (DE); COLBATZKY, Florian, 88441 Stafflangen (DE); PLATZ, Stefan, 88444 Ummendorf (DE)
(86) Internationale Anmeldenummer: PCT/EP2003/004606
(87) Internationale Veröffentlichungsnummer: WO 2003/094921

(56) Entgegenhaltungen:
- EP-A- 0 799 619
- EP-A- 0 799 619
- EP-A- 1 123 705
- EP-A- 1 123 705
- WO-A-02/18375
- WO-A-02/50043
- WO-A-02/50043
- WO-A-96/33980
- WO-A-96/33980
- DE-A- 10 017 539
- DE-A- 10 017 539
- DE-A- 10 042 060
- DE-A- 10 042 060
- DE-A- 10 042 064
- DE-A- 10 042 064
- DE-A- 19 825 591
- DE-A- 19 825 591
- US-A1- 2002 156 023
- BASELGA J ET AL: "EPITHELIAL GROWTH FACTOR RECEPTOR INTERACTING AGENTS" HEMATOLOGY - ONCOLOGY CLINICS OF NORTH AMERICA, W.B. SAUNDERS, US, Bd. 16, Nr. 5, Oktober 2002 (2002-10), Seiten 1041-1063, XP009052680 ISSN: 0889-8588

## Beschreibung

Gegenstand der vorliegenden Erfindung ist die Verwendung von EGF-Rezeptor Antagonisten, die ausgewählt sind aus der Gruppe bestehend aus
(1) 4-[(3-Chlor-4-fluorphenyl)amino]-6-{[4-(N,N-dimethylamino)-1-oxo-2-buten-1-yl]amino}-7-cyclopentyloxy-chinazolin,
(2) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin,
(3) 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin,
(4) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
(5) 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin,
(6) 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin,
deren Tautomeren, deren Stereoisomeren und deren Salzen,
zur Herstellung eines Arzneimittels zur Vorbeugung oder Behandlung von benigner (gutartiger) Prostatahyperplasie (BPH) und / oder Prostatahypertrophy,
gegebenenfalls in Kombination mit einer bereits bekannten Verbindung zur Behandlung von benigner Prostatahyperplasie / Prostatahypertrophy, ausgewählt aus der Gruppe (D) bestehend aus Osaterone, α1-Adrenorezeptor-Antagonisten, nicht-selektiven und selektiven Muskarin-Antagonisten, LHRH-/GnRH-Antagonisten oder Testosteron-5α-Reduktase-Hemmern.

Als α1-Adrenorezeptor-Antagonisten können in der erfindungsgemäßen Verwendung vorzugsweise die Wirkstoffe KMD-3231 (Silodosin), AIO-8507L, UK-338003, RBX-2258 (Parvosin), SNAP-6383 (L 771688), GYKI-16084, UK-294315, (S)-Doxazosin, Tamsulosin, Prazosin, Naftopidil, Terazosin, Alfuzosin oder Indoramin eingesetzt werden.

Als nicht selektive und selektive Muskarin-Antagonisten können in der erfindungsgemäßen Verwendung vorzugsweise die Wirkstoffe Darifenacin oder Tolterodine eingesetzt werden.

Als LHRH-/GnRH-Antagonisten können in der erfindungsgemäßen Verwendung vorzugsweise die Wirkstoffe Goserelin, Desorelin, Cetrorelix oder Gonadorelin eingesetzt werden.

Als Testosteron-5α-Reduktase-Hemmer können in der erfindungsgemäßen Verwendung vorzugsweise die Wirkstoffe GI-198745 (Dutasteride), LY-320236 (Izonsterid), TF 505, AS-601811, Finasteride, FK-687 oder CS-891 eingesetzt werden.

### Hintergrund der Erfindung

Benigne Prostatahyperplasie (BPH) / Prostatahypertrophy, eine gutartige Vergrößerung der Prostata, ist eine allgemein bekannte Störung bei Männern, die gewöhnlich ab einem Alter von 50 Jahren klinisch offenkundig zu Tage tritt. Etwa 50% aller Männer in einem Alter oberhalb von 50 Jahren und 95% aller Männer oberhalb von 70 Jahren sind davon betroffen.
Bei benigner Prostatahyperplasie / Prostatahypertrophy handelt es sich um einen allgemein fortschreitenden Zustand, der in schwerwiegenderen Fällen die Nierenfunktion gefährden kann und einen chirurgischen Eingriff erforderlich macht. Die Zahl der unbehandelten Patienten beläuft sich weltweit auf über 37 Millionen. Durch die Wucherung der Prostata wird die Harnröhre zusammengedrückt oder verlängert, was Symptome einer Harnleiterblockade verursacht und zum Urinrückstau führen kann.

Die Prostata besteht aus epithelialen Drüsenschläuchen, die in fibromuskuläres Stroma eingebettet sind. Das hyperplastische Wachstum der Prostata beginnt etwa im 30. Lebensjahr in den periurethral gelegenen Drüsenabschnitten, der sogenannten Übergangszone. Neben dem Einfluß des Alterns bilden androgene Hormone einen entscheidenden Wachstumsstimulus in der postpubertären Drüsenvolumenregulation. In der normalen Prostata wandelt das Enzym 5α-Reduktase in den Epithelzellen das androgene Hormon Testosteron (T) in Dihydrotestosteron (DHT) um. DHT, ein aktiver androgener prostatischer Metabolit, bindet an zytoplasmatische Rezeptoren und wird in den Zellkern transportiert, wo es RNA- und Proteinsynthese sowie die Zellreplikation einleitet. Es wird angenommen, dass sich BPH in Antwort auf die Einwirkung von DHT auf die alternde Prostata und auf Veränderungen in den Stroma- und Epithelzellen bildet (Steers, Zorn, Dis. Mon., 41 (7):437-497 (1995)).
Altersabhängige Veränderungen hinsichtlich der Serumkonzentrationen des gesamten hormonalen Regelkreises (LH, FSH, SHGB, T und DHT) sowie anderer Hormone, die auf diesen Regelkreis Einfluß nehmen können (Östrogene, Prolaktin, Testosteronderivate), sind als mögliche Ursache untersucht worden. Eine Korrelation zwischen solchen altersabhängigen Hormonveränderungen im Serum und den intraprostatischen Hormonkonzentrationen fehlt allerdings. Es wird somit deutlich, dass die Prostata für die Regulation des hormonellen Milieus selbst verantwortlich ist.
Mögliche Angriffspunkte für die Steuerung des intraprostatischen Hormonmilieus sind die 5α-Reduktase (also der Androgenmetabolismus), die Hormonrezeptorexpression in Epithel und Stroma, Östrogene und andere Hormone. Daneben beeinflussen zahlreiche peptidale Wachstumsfaktoren parakrin oder autokrin den lokalen Stoffwechsel in den verschiedenen Kompartimenten der Drüse, wodurch das Gleichgewicht der Zellkinetik zwischen Proliferation und programmiertem Zelltod verschoben werden kann.
Die klinischen Symptomen von BPH umfassen sowohl blockierende Symptome (z.B. Stocken des Harnstrahls, schwacher oder unterbrochener Strahl, Harnstauung), welche direkt aus der Einengung des Blasenhalses und der prostatischen Harnröhre durch die hyperplastische Prostata resultieren, als auch Symptome eines gereizten unteren Hamtraktes (z.B. Harnfrequenz, Nykturie, Dysurie, Hamdrang, Dranginkontinenz). Unbehandelt kann BPH zu schwerwiegenderen Komplikationen der Harnwege und Nieren führen, wie z.B. akute Harnstauung und Hydronephrose (Harnstauniere).

### Beschreibung der Erfindung

Die Aufgabe der vorliegenden Erfindung bestand in der Auffindung neuer Wirkstoffe zur Vorbeugung und/oder Behandlung von benigner Prostatahyperplasie / Prostatahypertrophy.
Überraschenderweise hat sich herausgestellt, dass die vorstehend erwähnten EGF-Rezeptor Antagonisten (1) bis (6) in besonderer Weise zur Vorbeugung und/oder Behandlung von benigner Prostatahyperplasie / Prostatahypertrophy geeignet sind, insbesondere jedoch die Verbindungen
(1) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin,
(2) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin oder
(3) 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin,
deren Tautomere, deren Stereoisomere oder deren Salze.

Weiterhin können auch Kombinationen der vorstehend genannten Wirkstoffe mit anderen, bereits auf dem Markt befindlichen Substanzen zur Behandlung von benigner Prostatahyperplasie / Prostatahypertrophy eingesetzt werden.

Ein weiterer Gegenstand der Erfindung ist somit die Verwendung eines der vorstehend erwähnten EGF-Rezeptor Antagonisten (1) bis (6) zur Herstellung eines Arzneimittels zur Vorbeugung oder Behandlung von benigner (gutartiger) Prostatahyperplasie (BPH) und / oder Prostatahypertrophy in Kombination mit einer therapeutisch wirksamen Menge eines anderen aktiven Arzneistoffes gegen benigne Prostatahyperplasie / Prostatahypertrophy (D) ausgewählt aus der Gruppe bestehend aus Osaterone, einem α1-Adrenorezeptor-Antagonisten, nicht-selektiven und selektiven Muskarin-Antagonisten, LHRH-/GnRH-Antagonisten und einem Testosteron-5α-Reduktase-Hemmer.

Der α1-Adrenorezeptor-Antagonist kann aus der Gruppe ausgewählt werden, die aus den Wirkstoffen KMD-3231 (Selodosin), AIO-8507L, UK-338003, RBX-2258 (Parvosin), SNAP-6383 (L 771688), GYKI-16084, UK-294315, (S)-Doxazosin, Tamsulosin, Prazosin, Naftopidil, Terazosin, Alfuzosin und Indoramin besteht.

Der nicht-selektive oder selektive Muskarin-Antagonist kann aus der Gruppe ausgewählt werden, die aus den Wirkstoffen Darifenacin und Tolterodine besteht.

Der LHRH-/GnRH-Antagonist kann aus der Gruppe ausgewählt werden, die aus den Wirkstoffen Goserelin, Desorelin, Cetrorelix und Gonadorelin besteht.

Der Testosteron-5α-Reduktase-Hemmer kann aus der Gruppe ausgewählt werden, die aus den Wirkstoffen GI-198745 (Dutasteride), LY-320236 (Izonsterid), TF 505, AS-601811, Finasteride, FK-687 und CS-891 besteht.

In einer bevorzugten Ausführungsform der erfindungsgemäßen Verwendung wird der Arzneistoff (D) aus der Gruppe ausgewählt, die aus Tamsulosin, UK-338003, Terazosin, Indoramin, Tolterodine, Dutasteride und Finasteride besteht.

In einer besonders bevorzugten Ausführungsform des erfindungsgemäßen Verwendung wird Tamsulosin verwendet.

Die Dosierung des vorstehend erwähnten EGF-Rezeptor Antagonisten (1) bis (6) beträgt 0.01 mg bis 300 mg bis zu 3mal täglich.

Die Dosierung der Komponente (D) für die Arzneimittelkombination ist ungefähr 1/10 der niedrigsten normalerweise empfohlenen Dosis bis zu 1/1 der normalerweise empfohlenen Dosis.

Ein vorstehend erwähnter EGF-Rezeptor Antagonist (1) bis (6) kann beispielsweise in Kombination mit

Tamsulosin, das auf oralem Weg in einer Dosierung von 0.2 bis 0.8 mg einmal täglich verabreicht werden kann, oder in Kombination mit
Naftopidil, das auf oralem Weg in einer Dosierung von 25 bis 100 mg pro Tag verabreicht werden kann, oder in Kombination mit
Terazosin, das auf oralem Weg in einer Dosierung von 1 bis 20 mg pro Tag verabreicht werden kann, oder in Kombination mit
Alfuzosin, das auf oralem Weg in einer Dosierung von dreimal täglich 2.5 mg bis einmal täglich 10 mg verabreicht werden kann, oder in Kombination mit
Indoramin, das auf oralem Weg in einer Dosierung von 50 bis 200 mg pro Tag verabreicht werden kann, oder in Kombination mit
Doxazosin, das auf oralem Weg in einer Dosierung von 1 bis 16 mg pro Tag verabreicht werden kann, oder in Kombination mit
Dutasteride, das auf oralem Weg in einer Dosierung von 0.5 mg pro Tag verabreicht werden kann, oder in Kombination mit
Finasteride, das auf oralem Weg in einer Dosierung von 1 bis 5 mg pro Tag verabreicht werden kann, oder in Kombination mit
Tolterodine, das auf oralem Weg in einer Dosierung von 2 bis 4 mg pro Tag verabreicht werden kann, zur Behandlung von benigner Prostatahyperplasie /Prostatahypertrophy verwendet werden.

Weiterhin kann eine erfindungsgemäße pharmazeutische Zusammensetzung ein Kit von Teilen für die Behandlung und/oder Prävention von benigner Prostatahyperplasie / Prostatahypertrophy sein, wobei das Kit umfaßt:
(a) eine erste Umschließung enthaltend eine pharmazeutische Zusammensetzung umfassend eine therapeutisch wirksame Menge eines Wirkstoffs ausgewählt aus vorstehend erwähnten EGF-Rezeptor Antagonisten (1) bis (6) und ein oder mehrere pharmazeutisch verträgliche Verdünnungsmittel und/oder Träger; und
(b) eine zweite Umschließung enthaltend eine pharmazeutische Zusammensetzung umfassend einen der Arzneistoffe (D) und einen oder mehrere pharmazeutisch verträgliche Verdünnungsmittel und/oder Träger.

Ein bevorzugtes Kit umfaßt Tamsulosin in der zweiten Umschließung.

Die Wirkstoffe können oral oder rektal verabreicht werden.

Zur Verabreichung können übliche Darreichungsformen verwendet werden. Beispielsweise lassen sich die Wirkstoffe, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/- Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die Wirkstoffe können oral in einer breiten Vielfalt von verschiedenen Dosierungsformen verabreicht werden, beispielsweise können sie zusammen mit verschiedenen pharmazeutisch annehmbaren inerten Trägern in Form von Tabletten, Kapseln, Pastillen, Plätzchen, harten Bonbons, Pulvern, wässrigen Suspensionen, Elixiren, Sirupen und dergleichen formuliert werden. Derartige Träger umfassen beispielsweise feste Verdünner oder Füllstoffe, sterile wässrige Medien und verschiedene nichttoxische organische Lösungsmittel. Zudem können derartige orale Formulierungen auf geeignete Weise mit Hilfe von verschiedenen, üblicherweise für diesen Zweck eingesetzten Agenzien gesüßt und/oder aromatisiert sein. Im allgemeinen sind die Wirkstoffe in solchen oralen Dosierungsformen mit Konzentrationsmengen vorhanden, deren Bereich, bezogen auf die Gesamtzusammensetzung, von etwa 0.5 Gew.-% bis etwa 90 Gew.-% reicht, in Mengen, die ausreichen, um die gewünschten Dosierungseinheiten zu ergeben. Andere geeignete Dosierungsformen für die Wirkstoffe umfassen Formulierungen zur kontrollierten Freisetzung und Vorrichtungen, die den Fachpersonen auf dem betreffenden Gebiet wohlbekannt sind.

Wie bereits eingangs erwähnt, weisen die vorstehend genannten Verbindungen (1) bis (6) und deren Salze wertvolle Eigenschaften auf. Insbesondere sind sie zur Vorbeugung und/oder Behandlung von benigner Prostatahyperplasie /Prostatahypertrophy geeignet.

Beispielsweise wurden die folgenden Verbindungen

| | |
|---|---|
| Verbindung a = | 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin, |
| Verbindung b = | 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin und |
| Verbindung c = | 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin |

auf ihre prostata-verkleinernde Wirkung getestet.

### Experimenteller Teil

### 1) Versuchsdurchführung:

Drei Gruppen von jeweils fünf männlichen Ratten (ca. 7 Wochen alt und etwa 185 g schwer) werden 14-15 Tage lang mit einem EGF-R-TK Inhibitor behandelt. Die Einbringung des Wirkstoffs erfolgt als Suspension über eine Schlundsonde einmal pro Tag. Nach Ende der Versuchsdauer werden die Tiere getötet, die Prostata entnommen und gewogen.
Die Ergebnisse der Versuchsreihen sind in den nachstehend angeführten Tabellen aufgelistet.

### 2) Versuchsergebnisse:

**Tabelle 1: Verabreichung von Verbindung a (Behandlungszeitraum: 14 Tage)**

| **Dosierungsgruppe** | **Kontrolle** | **Verbindung a** | | |
|---|---|---|---|---|
| Dosierungshöhe | 0 mg/kg | 10 mg/kg | 30 mg/kg | 100 mg/kg |
| durchschnittliches Absolutgewicht [g] | 0.4641 | 0.4100 | 0.3419^{*} | 0.1099^{*} |
| durchschnittliches relatives Gewicht [% des Körpergewichts] | 0.1913 | 0.1718 | 0.1473^{*} | 0.0672^{*} |
| durchschnittliches relatives Gewicht [% des Gehirns] | 23.64 | 20.62 | 17.07^{*} | 5.55^{*} |

| | | | | |
|---|---|---|---|---|
| * p < 0.05 | | | | |

**Tabelle 2: Verabreichung von Verbindung b (Behandlungszeitraum: 14 Tage)**

| **Dosierungsgruppe** | **Kontrolle** | **Verbindung b** | | |
|---|---|---|---|---|
| Dosierungshöhe | 0 mg/kg | 10 mg/kg | 30 mg/kg | 100 mg/kg |
| durchschnittliches Absolutgewicht [g] | 0.4641 | 0.3983^{*} | 0.3989 | 0.1848^{*} |
| durchschnittliches relatives Gewicht [% des Körpergewichts] | 0.1913 | 0.1622^{*} | 0.1697 | 0.1191^{*} |
| durchschnittliches relatives Gewicht [% des Gehirns] | 23.64 | 20.23^{*} | 20.71^{*} | 10.13^{*} |

| | | | | |
|---|---|---|---|---|
| * p < 0.05 | | | | |

**Tabelle 3: Verabreichung von Verbindung c (Behandlungszeitraum: 15 Tage)**

| **Dosierungsgruppe** | **Kontrolle** | **Verbindung c** | | |
|---|---|---|---|---|
| Dosierungshöhe | 0 mg/kg | 10 mg/kg | 30 mg/kg | 100 mg/kg |
| durchschnittliches Absolutgewicht [g] | 0.4641 | 0.3854^{*} | 0.2242^{*} | 0.1463^{*} |
| durchschnittliches relatives Gewicht [% des Körpergewichts] | 0.1913 | 0.1681 | 0.1117^{*} | 0.0892^{*} |
| durchschnittliches relatives Gewicht [% des Gehirns] | 23.64 | 19.67^{*} | 11.50^{*} | 7.63^{*} |

| | | | | |
|---|---|---|---|---|
| * p < 0.05 | | | | |

Die vorstehend genannten Verbindungen, deren Herstellung nicht bereits im Stand der Technik beschrieben ist, werden nach folgendem Verfahren erhalten:

### Referenz-Beispiel 1

### 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin

Zu einer Lösung aus 4.50 g Bromcrotonsäure in 60 ml Methylenchlorid werden 4.70 ml Oxalylchlorid getropft. Anschließend wird ein Tropfen N,N-Dimethylformamid zugegeben. Nach ca. 30 Minuten ist die Gasentwicklung beendet und das Reaktionsgemisch wird am Rotationsverdampfer eingeengt. Das rohe Bromcrotonsäurechlorid wird in 30 ml Methylenchlorid aufgenommen und unter Eisbad-Kühlung zu einer Lösung aus 7.00 g 4-[(3-Chlor-4-fluorphenyl)amino]-6-amino-7-cyclopropyl-methoxy-chinazolin und 10.20 ml Hünigbase in 150 ml Tetrahydrofuran getropft. Das Reaktionsgemisch wird etwa 1.5 Stunden unter Eisbadkühlung und weitere zwei Stunden bei Raumtemperatur gerührt. Nun werden 5.20 g N-(2-Methoxy-ethyl)-N-methylamin zugegeben und das Reaktionsgemisch wird über Nacht bei Raumtemperatur gerührt. Zur Aufarbeitung es wird mit Methylenchlorid verdünnt und gründlich mit Wasser gewaschen. Die organische Phase wird über Magnesiumsulfat getrocknet und eingeengt. Das Rohprodukt wird chromatographisch über eine Kieselgelsäule mit Essigester gefolgt von Essigester/Methanol (19:1) als Laufmittel gereinigt.
Ausbeute: 5.07 g (51 % der Theorie)
Massenspektrum (ESI⁻): m/z = 512, 514 [M-H]⁻
R_{f}-Wert: 0.25 (Kieselgel, Essigester/Methanol = 9:1)

Analog Referenz-Beispiel 1 werden folgende Verbindungen erhalten:
(1) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin,
(2) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, und
(3) 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin.

## Patentansprüche

1. Verwendung der EGF-Rezeptor Antagonisten ausgewählt aus der Gruppe bestehend aus
(1) 4-[(3-Chlor-4-fluorphenyl)amino]-6-{[4-(N,N-dimethylamino)-1-oxo-2-buten-1-yl]amino}-7-cyclopentyloxy-chinazolin,
(2) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin,
(3) 4-[(3-Chlor-4-fluorphenyl)amino]-6-{[4-(N,N-dimethylamino)-1-oxo-2-buten-1-yl]amino}-7-((*R*)-tetrahydrofuran-3-yloxy)-chinazolin,
(4) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
(5) 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin,
(6) 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin,
deren Tautomeren, deren Stereoisomeren und deren Salzen,
zur Herstellung eines Arzneimittels zur Vorbeugung oder Behandlung von benigner (gutartiger) Prostatahyperplasie (BPH) und / oder Prostatahypertrophy.

2. Verwendung der EGF-Rezeptor Antagonisten gemäß Anspruch 1, **dadurch gekennzeichnet, dass** diese ausgewählt sind aus der Gruppe bestehend aus
(1) 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin,
(2) 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin ,
(3) 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin,
deren Tautomeren, deren Stereoisomeren und deren Salzen.

3. Verwendung eines Wirkstoffs gemäß einem der Ansprüche 1 oder 2 in Kombination mit einer therapeutisch wirksamen Menge eines anderen aktiven Arzneistoffs (D) gegen benigne Prostatahyperplasie / Prostatahypertrophy, ausgewählt aus der Gruppe bestehend aus Osaterone, α1-Adrenorezeptor-Antagonisten, nicht-selektiven und selektiven Muskarin-Antagonisten, LHRH-/GnRH-Antagonisten und Testosteron-5α-Reduktase-Hemmern, zur Herstellung eines Arzneimittels zur Prävention und/oder Behandlung von benigner Prostatahyperplasie / Prostatahypertrophy.

4. Verwendung gemäß Anspruch 3, **dadurch gekennzeichnet, dass** der α1-Adrenorezeptor-Antagonist ausgewählt ist aus der Gruppe bestehend aus den Wirkstoffen KMD-3231 (Silodosin), AIO-8507L, UK-338003, RBX-2258 (Parvosin), SNAP-6383 (L 771688), GYKI-16084, UK-294315, (S)-Doxazosin, Tamsulosin, Prazosin, Naftopidil, Terazosin, Alfuzosin und Indoramin.

5. Verwendung gemäß Anspruch 3, **dadurch gekennzeichnet, dass** der unselektive oder selektive Muskarin-Antagonist ausgewählt ist aus der Gruppe bestehend aus den Wirkstoffen Darifenacin und Tolterodine.

6. Verwendung gemäß Anspruch 3, **dadurch gekennzeichnet, dass** der LHRH-/GnRH-Antagonist ausgewählt ist aus der Gruppe bestehend aus den Wirkstoffen Goserelin, Desorelin, Cetrorelix und Gonadorelin.

7. Verwendung gemäß Anspruch 3, **dadurch gekennzeichnet, dass** der Testosteron-5α-Reduktase-Hemmer ausgewählt ist aus der Gruppe bestehend aus den Wirkstoffen GI-198745 (Dutasterid), LY-320236 (Izonsterid), TF 505, AS-601811, Finasteride, FK-687 und CS-891.

8. Verwendung gemäß Anspruch 3, **dadurch gekennzeichnet, dass** der Arzneistoff (D) ausgewählt ist aus der Gruppe bestehend aus Tamsulosin, UK-338003, Terazosin, Indoramin, Tolterodine, Dutasteride und Finasteride.

9. Verwendung gemäß Anspruch 3, **dadurch gekennzeichnet, dass** der Arzneistoff (D) Tamsulosin ist.

10. Kit von Teilen für die Behandlung und/oder Prävention von benigner Prostatahyperplasie / Prostatahypertrophy, wobei das Kit umfaßt:
(a) eine erste Umschließung enthaltend eine pharmazeutische Zusammensetzung umfassend eine therapeutisch wirksame Menge eines Wirkstoffs gemäß Anspruch 1 und ein oder mehrere pharmazeutisch verträgliche Verdünnungsmittel und/oder Träger; und
(b) eine zweite Umschließung enthaltend eine pharmazeutische Zusammensetzung umfassend einen der Arzneistoffe (D) gemäß Anspruch 3 und einen oder mehrere pharmazeutisch verträgliche Verdünnungsmittel und/oder Träger.

11. Kit von Teilen gemäß Anspruch 10, **dadurch gekennzeichnet, dass** der Arzneistoff (D) Tamsulosin ist.

## Claims

1. Use of the EGF-receptor antagonists selected from the group consisting of
(1) 4-[(3-chloro-4-fluorophenyl)amino]-6-{[4-(N,N-dimethylamino)-1-oxo-2-buten-1-yl]amino}-7-cyclopentyloxy-quinazoline,
(2) 4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline,
(3) 4-[(3-chloro-4-fluorophenyl)amino]-6-{[4-(N,N-dimethylamino)-1-oxo-2-buten-1-yl]amino}-7-((*R*)-tetrahydrofuran-3-yloxy)-quinazoline,
(4) 4-[(3-chloro-4-fluorophenyl)amino]-6-{[4-(N-cyclopropyl-N-methyl-amino)-1-oxo-2-buten-1-yl]amino}-7-cyclopentyloxy-quinazoline,
(5) 4-[(3-chloro-4-fluorophenyl)amino]-6-{[4-(N,N-dimethylamino)-1-oxo-2-buten-1-yl]amino}-7-[(*R*)-(tetrahydrofuran-2-yl)methoxy]-quinazoline,
(6) 4-[(3-chloro-4-fluorophenyl)amino]-6-{[4-(N,N-dimethylamino)-1-oxo-2-buten-1-yl]amino}-7-[(*S*)-(tetrahydrofuran-2-yl)methoxy]-quinazoline,
or the tautomers, the stereoisomers and the salts thereof,
for preparing a medicament for the prevention or treatment of benign prostatic hyperplasia (BPH) and / or prostatic hypertrophy.

2. Use of the EGF-receptor antagonists according to claim 1, **characterised in that** they are selected from the group consisting of
(1) 4-[(3-chloro-4-fluorophenyl)amino]-6-{[4-(N,N-dimethylamino)-1-oxo-2-buten-1-yl]amino}-7-((*S*)-tetrahydrofuran-3-yloxy)-quinazoline,
(2) 4-[(3-chloro-4-fluorophenyl)amino]-6-{[4-(N-cyclopropyl-N-methyl-amino)-1-oxo-2-buten-1-yl]amino}-7-cyclopentyloxy-quinazoline,
(3) 4-[(3-chloro-4-fluorophenyl)amino]-6-{[4-(N,N-dimethylamino)-1-oxo-2-buten-1-yl]amino}-7-[(*S*)-(tetrahydrofuran-2-yl)methoxy]-quinazoline,
or the tautomers, stereoisomers and salts thereof.

3. Use of an active substance according to one of claims 1 or 2, in combination with a therapeutically effective amount of another medicament (D) active against benign prostatic hyperplasia / prostatic hypertrophy, selected from the group consisting of osaterone, α1-adrenoreceptor antagonists, non-selective and selective muscarine antagonists, LHRH/GnRH antagonists and testosterone-5α-reductase inhibitors, for preparing a medicament for the prevention and/or treatment of benign prostatic hyperplasia / prostatic hypertrophy.

4. Use according to claim 3, **characterised in that** the α1-adrenoreceptor antagonist is selected from the group consisting of the active substances KMD-3231 (silodosin), AIO-8507L, UK-338003, RBX-2258 (parvosin), SNAP-6383 (L 771688), GYKI-16084, UK-294315, (S)-doxazosin, tamsulosin, prazosin, naftopidil, terazosin, alfuzosin and indoramin.

5. Use according to claim 3, **characterised in that** the non-selective or selective muscarine antagonist is selected from the group consisting of the active substances darifenacin and tolterodine.

6. Use according to claim 3, **characterised in that** the LHRH/GnRH antagonist is selected from the group consisting of the active substances goserelin, desorelin, cetrorelix and gonadorelin.

7. Use according to claim 3, **characterised in that** the testosterone-5α-reductase inhibitor is selected from the group consisting of the active substances GI-198745 (dutasteride), LY-320236 (izonsteride), TF 505, AS-601811, finasteride, FK-687 and CS-891.

8. Use according to claim 3, **characterised in that** the medicament (D) is selected from the group consisting of tamsulosin, UK-338003, terazosin, indoramin, tolterodine, dutasteride and finasteride.

9. Use according to claim 3, **characterised in that** the medicament (D) is tamsulosin.

10. Kit of parts for the treatment and/or prevention of benign prostatic hyperplasia / prostatic hypertrophy, this kit comprising:
(a) a first container containing a pharmaceutical composition comprising a therapeutically effect amount of an active substance according to claim 1 and one or more pharmaceutically acceptable diluents and/or carriers; and
(b) a second container containing a pharmaceutical composition comprising one of the medicaments (D) according to claim 3 and one or more pharmaceutically acceptable diluents and/or carriers.

11. Kit of parts according to claim 10, **characterised in that** the medicament (D) is tamsulosin.

## Revendications

1. Utilisation d'antagonistes du récepteur EGF choisi dans le groupe comprenant
(1) la 4-[(3-chloro-4-fluorophényl)amino]6-{[4-(N,N-diméthylamino)-1-oxo-2-butén-1-yl]amino}-7-cyclopentyloxy-quinazoline,
(2) la 4-[(3-chloro-4-fluorophényl)amino]6-{[4-(N,N-diméthylamino)-1-oxo-2-butén-1-yl]amino}-7-((S)-tétrahydrofuran-3-yloxy)-quinazoline,
(3) la 4-[(3-chloro-4-fluorophényl)amino]6-{[4-(N,N-diméthylamino)-1-oxo-2-butén-1-yl]amino}-7-((R)-tétrahydrofuran-3-yloxy)-quinazoline,
(4) la 4-[(3-chloro-4-fluorophényl)amino]6-{[4-(N-cyclopropyl-N-méthyl-amino)-1-oxo-2-butén-1-yl]amino}-7-cyclopentyloxy-quinazoline,
(5) la 4-[(3-chloro-4-fluorophényl)amino]6-{[4-(N,N-diméthylamino)-1-oxo-2-butén-1-yl]amino}-7-[(R)-tétrahydrofuran-2-yl)méthoxy]-quinazoline,
(6) la 4-[(3-chloro-4-fluorophényl)amino]6-{[4-(N,N-diméthylamino)-1-oxo-2-butén-1-yl]amino}-7-[(S)-tétrahydrofuran-2-yl)méthoxy]-quinazoline,
leurs tautomères, leurs stéréo-isomères et leurs sels,
pour la production d'un médicament pour la prévention ou le traitement de l'hyperplasie prostatique (BPH) bénigne et/ou de l'hypertrophie prostatique.

2. Utilisation des antagonistes du récepteur de l'EGF selon la revendication 1, **caractérisée en ce que** ceux-ci sont choisis dans le groupe comprenant
(1) la 4-[(3-chloro-4-fluorophényl)amino]6-{[4-(N,N-diméthylamino)-1-oxo-2-butén-1-yl]amino}-7-((S)-tétrahydrofuran-3-yloxy)-quinazoline,
(2) la 4-[(3-chloro-4-fluorophényl)amino]-6-{[4-(N-cyclopropyl-N-méthyl-amino)-1-oxo-2-butén-1-yl]amino}-7-cyclopentyloxy-quinazoline,
(3) la 4-[(3-chloro-4-fluorophényl)amino]6-{[4-(N,N-diméthylamino)-1-oxo-2-butén-1-yl]amino}-7-[(S)-tétrahydrofuran-2-yl)méthoxy]-quinazoline,
leurs tautomères, leurs stéréo-isomères et leurs sels.

3. Utilisation d'une substance active selon l'une des revendications 1 ou 2, en combinaison avec une quantité thérapeutiquement efficace d'un autre médicament actif (D) contre une hyperplasie prostatique bénigne /hypertrophie prostatique, choisie dans le groupe comprenant l'osatérone, les antagonistes de l'adrénorécepteur α1, les antagonistes non sélectifs et sélectifs de la muscarine, les antagonistes de la LHRH/GnRH et les inhibiteurs de la testostérone-5α-réductase, pour la production d'un médicament pour la prévention et/ou le traitement de l'hyperplasie prostatique bénigne / l'hypertrophie prostatique.

4. Utilisation selon la revendication 3, **caractérisée en ce que** l'antagoniste de l'adrénorécepteur α1 est choisi dans le groupe comprenant les substances actives KMD-3231 (silodosine), AIO-8507L, UK-338003, RBX-2258 (parvosine), SNAP-6383 (L 771688), GYKI-16084, UK-294315, (S)-doxazosine, tamsulosine, prazosine, naftopidil, térazosine, alfuzosine et indoramine.

5. Utilisation selon la revendication 3, **caractérisée en ce que** l'antagoniste non sélectif ou sélectif de la muscarine est choisi dans le groupe comprenant les substances actives darifénacine ou toltérodine.

6. Utilisation selon la revendication 3, **caractérisée en ce que** l'antagoniste de la LHRH/GnRH est choisi dans le groupe comprenant les substances actives goséréline, désoréline, celtrorelix et gonadoréline.

7. Utilisation selon la revendication 3, **caractérisée en ce que** l'inhibiteur de la testostérone-5α-réductase est choisi dans le groupe comprenant les substances actives GI-198745 (dutastéride), LY-320236 (izonstéride), TF 505, AS-601811, finastéride, FK-687 et CS-891.

8. Utilisation selon la revendication 3, **caractérisée en ce que** le médicament (D) est choisi dans le groupe comprenant la tamsulosine, l'UK-338003, la térazosine, l'indoramine, la toltérodine, le dutastéride et le finastéride.

9. Utilisation selon la revendication 3, **caractérisée en ce que** le médicament (D) est la tamsulosine.

10. Kit d'éléments pour le traitement et/ou la prévention de l'hyperplasie prostatique bénigne / de l'hypertrophie prostatique, le kit contenant :
(a) un premier emballage contenant une composition pharmaceutique comprenant une quantité thérapeutiquement efficace d'une substance active selon la revendication 1 et un ou plusieurs diluants et/ou véhicules pharmaceutiquement acceptables ; et
(b) un deuxième emballage contenant une composition pharmaceutique comprenant l'une des substances actives (D) selon la revendication 3 et un ou plusieurs diluants et/ou véhicules pharmaceutiquement acceptables.

11. Kit d'éléments selon la revendication 10, **caractérisé en ce que** le médicament (D) est la tamsulosine.
